(19) Europäisches Patentamt
European Patent Office
Office européen des brevets

(11) **EP 3 789 911 B1**

(12) **EUROPEAN PATENT SPECIFICATION**

(45) Date of publication and mention
of the grant of the patent:
**29.05.2024  Bulletin 2024/22**

(51) International Patent Classification (IPC):
*A61B 5/283* (2021.01)    *G06N 3/08* (2023.01)
*A61B 18/14* (2006.01)   *A61B 18/00* (2006.01)
*A61B 5/35* (2021.01)    *A61B 5/367* (2021.01)

(21) Application number: **19792573.8**

(52) Cooperative Patent Classification (CPC):
**A61B 18/1206;** A61B 18/1492; A61B 2018/00357;
A61B 2018/00577; A61B 2018/00839

(22) Date of filing: **24.04.2019**

(86) International application number:
**PCT/CN2019/084012**

(87) International publication number:
**WO 2019/206163 (31.10.2019 Gazette 2019/44)**

(54) **ABLATION FOCUS EVALUATION SYSTEM**

SYSTEM ZUR ABLATIONSFOKUSBEURTEILUNG

SYSTÈME D'ÉVALUATION DE FOYER D'ABLATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2018  CN 201810402737**

(43) Date of publication of application:
**10.03.2021  Bulletin 2021/10**

(73) Proprietor: **Shanghai Microport Ep Medtech Co.,
Ltd.
Shanghai 201318 (CN)**

(72) Inventors:
• **CHU, Huimin**
  **Shanghai 201318 (CN)**
• **CHENG, Huasheng**
  **Shanghai 201318 (CN)**
• **ZHANG, Qingchun**
  **Shanghai 201318 (CN)**
• **PENG, Yahui**
  **Shanghai 201318 (CN)**
• **SHEN, Liuping**
  **Shanghai 201318 (CN)**
• **SUN, Yiyong**
  **Shanghai 201318 (CN)**

(74) Representative: **Patentship
Patentanwaltsgesellschaft mbH
Paul-Gerhardt-Allee 50
81245 München (DE)**

(56) References cited:
EP-A1- 3 212 074       EP-B1- 3 212 074
CN-A- 103 190 951      CN-A- 103 519 884
CN-A- 105 050 521      CN-A- 107 148 249
CN-A- 107 582 042      CN-A- 108 596 132
US-A1- 2004 059 237

**Description**

**TECHNICAL FIELD**

The present invention relates to the field of medical equipments and, in particular, to an ablation lesion assessment system.

**BACKGROUND**

Cardiac arrhythmia may occur as an abnormal rate and/or rhythm of the heartbeat due to the conduction of an abnormal sinoatrial (SA) node impulse or an impulse originating outside the SA node, which is delayed or blocked or takes place in an irregular pathway, i.e., an abnormality in the generation and/or propagation of cardiac electrical activity. For instance, atrial fibrillation (AF) is a tachycardia arrhythmia caused by the abnormal electrical signals.

Cardiac radiofrequency (RF) ablation has been widely used in the treatment of cardiac arrhythmias. This therapy utilizes various levels of energy to eliminate the source of an abnormal electrical signal by ablating the tissue that generates the signal, cutting off its pathway or destroying the abnormal tissue.

Cardiac radiofrequency ablation often leads to an ablated tissue region with transmural cell necrosis. Traditionally, for different lesion sites to be ablated, surgeons could determine, based on their experience, different values of the principal cardiac radiofrequency ablation parameters including RF power, ablation temperature and ablation time that control how the ablation carries on. However, this surgery remains risky for patients with complicated or unusual cardiac anatomy.

AF treatment is one important application of cardiac radiofrequency ablation. The pulmonary veins (PVs) have been widely recognized as AF trigger sites, and cardiac radiofrequency ablation for AF treatment, called pulmonary vein isolation (PVI), aims to ensure that there is not any single electrical connection between the pulmonary veins and the left atrium through electrically isolating triggers in the PVs.

PVI requires a continuous ablation lesion circumferentially around a pulmonary vein. However, since the PVs are complicated in anatomy and varying in wall thickness, ablation control is particularly critical for such procedures. Studies have found that electrical re-connection that may lead to AF recurrence or an iatrogenic atrial arrhythmia may happen, when effective isolation of the circumferential ablation lesion is not attained, e.g., with a tiny gap as small as 1 mm in the resulting scarline around the pulmonary vein. On the contrary, the application of excessive ablation energy may bring damage to other organs such as the esophagus and phrenic nerve, or even cause cardiac perforation.

Therefore, there is a need for a method or apparatus that allows a higher success rate and improved safety of cardiac radiofrequency ablation procedures through real-time acquisition of ablation information without reliance on the surgeons' experience.

EP 3 212 074 A1 discloses systems and methods for activating transducers to ablate tissue and providing information related to the tissue ablation. US 2004/059237 A1 discloses systems and methods for analyzing the electrocardiogram, pacing and mapping the heart for the diagnosis and treatment of cardiac conditions.

**SUMMARY OF THE INVENTION**

The present invention is set out in the appended set of claims. No method falls within the scope of the claims.

The present invention aims to obtain, in real time, during a cardiac radiofrequency ablation procedure, information about whether complete ablation has been achieved, so as to allow the surgeon to know how the ablation proceeds in a timely manner and accordingly perform better control over the applied ablation energy, which can result in a higher success rate and increased safety of the procedure.

According to one aspect of the present invention, there is provided an ablation lesion assessment system according to claim **1**.

Optionally, the intracardiac electrode signal may vary over time, wherein the signal processing module is configured to obtain and process the intracardiac electrode signal in real time at a predetermined refresh rate and thereby generate a corresponding real-time signal profile. The refresh rate may be 1-2000 Hz.

Optionally, when the ablation information indicates that the complete ablation has been achieved, the determining unit may be configured to output the ablation information with a delay of a predetermined period of time after the determination is made. The predetermined period of time may be 1-10 seconds.

Optionally, when the proportion is equal to 100%, the ablation information may indicate that the complete ablation has been achieved, otherwise indicate that complete ablation has not been achieved. Alternatively, when the waveform comparison result indicates a similarity, the ablation information may indicate that complete ablation has been achieved, otherwise indicate that complete ablation has not been achieved.

Optionally, when the proportion is equal to 100%, the waveform comparison unit feeds back the waveform comparison result to the determining unit, and when the waveform comparison unit indicates a similarity, the ablation information indicates that the complete ablation has been achieved, otherwise indicates that the complete ablation has not been achieved.

Optionally, the waveform comparison unit may be configured to calculate a degree of waveform similarity between the signal profile and the ablation pattern associated with the ablation lesion, wherein when the degree of waveform similarity is greater than a predetermined threshold, the waveform comparison result indicates the similarity and otherwise indicates a dissimilarity.

Optionally, the ablation lesion may be associated with a plurality of ablation patterns, and the waveform comparison unit is configured to calculate Pearson a correlation coefficient between the signal profile and each of the plurality of ablation patterns and obtain an overall Pearson correlation coefficient by taking an average or a median of a plurality of Pearson correlation coefficients, wherein when the overall Pearson correlation coefficient is 0.6-1, the waveform comparison result indicates the similarity, otherwise indicates a dissimilarity, and wherein each of the plurality of Pearson correlation coefficients is calculated according to

$$P(X,Y) = \frac{Cov(X,Y)}{\sigma_X \sigma_Y}$$

, where P (X, Y) represents the Pearson correlation coefficient, X and Y denote X and Y coordinates of the signal profile and the ablation pattern, $Cov(X,Y)$ is a covariance of X and Y, and $\sigma_X$ and $\sigma_Y$ are standard deviations of X and Y

Optionally, the ablation lesion may be associated with a plurality of ablation patterns, and the waveform comparison unit is configured to obtain the waveform comparison result by comparing the signal profile with the plurality of ablation patterns using a neural network algorithm.

Optionally, the ablation lesion assessment system may further comprise one or more of: an instruction reception module configured to activate or deactivate the ablation lesion assessment system and to set parameters of the ablation lesion assessment system; a signal input module configured to obtain and transmit the intracardiac electrode signal to the signal processing module; a display module and transmit display the ablation information; a voice module and transmit convey the ablation information; a storage module and transmit store the ablation pattern; and a pattern selection module and transmit determine an intracardiac site where the ablation lesion is being formed and to select the associated ablation pattern.

Optionally, the intracardiac electrode signal may be a single-electrode signal obtained by one electrode that is attached to an ablation catheter or an intracardiac mapping catheter and is brought into contact with the ablation lesion.

The ablation lesion assessment system provided in the present invention is able to analyze, during a cardiac radiofrequency ablation procedure, an intracardiac electrode signal from an electrode brought into contact with an ablation lesion and thereby obtain ablation information indicative of whether complete ablation has been achieved. This ablation information can be provided to a surgeon so that he/she can know in real time the progress of ablation, e.g., whether the tissue has been transmural (i.e., complete ablation has been achieved). In this way, the cardiac radiofrequency ablation can be conducted in a more accurate manner. For example, in a PVI procedure, the ablation lesion assessment system can facilitate the formation of a circumferential ablation lesion without gaps or incompletely ablated portions. Moreover, even after the ablation process, the ablation lesion assessment system can be used to identify any gap in a timely manner and allows re-ablation. Thus, a significantly higher success rate and greatly increased safety of cardiac radiofrequency ablation can be obtained.

The ablation lesion assessment system provided in the present invention can be implemented by a software program deployed outside the patient's body. The software program is stored in a computer readable medium and is applicable to any device or instrument that can display the intracardiac electrode signal, such as a multi-channel electrophysiological recorder, a three-dimensional mapping system or a radiofrequency ablation system. The electrode can be attached to a catheter, such as an ablation catheter or a mapping catheter, which has a distal end assuming a linear or annular shape or provided with a balloon. Ablation can be accomplished by RF energy, laser or freezing.

The ablation lesion assessment system provided in the present invention can also be implemented by a programmed computer system including a software program. Ablation information obtained, when the software program is executed, can help a surgeon more accurately control a cardiac radiofrequency ablation procedure so that a higher success rate and increased safety of the procedure can be achieved.

BRIEF DESCRIPTION OF THE DRAWINGS

Fig. 1 schematically illustrates an electrode brought into contact with an ablation lesion in accordance with an embodiment of the present invention.

Fig. 2A is a graphical representation of an intracardiac electrode signal from an incompletely ablated lesion in accordance with an embodiment of the present invention.

Fig. 2B is a graphical representation of an intracardiac electrode signal from a completely ablated lesion in accordance with an embodiment of the present invention.

Fig. 3 is a schematic illustration of pulmonary vein isolation (PVI).

Fig. 4 is a diagram illustrating the structure of an ablation lesion assessment system in accordance with an embodiment of the present invention.

Figs. 5(a) to 5 (d) are graphical representations of a signal profile in an ablation process in accordance with an embodiment of the present invention.

Fig. 6 schematically depicts a comparison drawn between a signal profile and an ablation pattern in accordance with an embodiment of the present invention.

Fig. 7 schematically illustrates ablation lesions for different intracardiac sites and corresponding ablation patterns in accordance with embodiments of the present invention.

Fig. 8 is a flowchart of an ablation lesion assessment method not forming part of the claims.

Fig. 9 schematically illustrates the use of the method in an ablation process not forming part of the claims.

In the figures,
20: an ablation lesion; 21: a transmural ablation lesion; 22: a nontransmural ablation lesion; 30: a pulmonary vein; 100: an ablation lesion assessment system; 110: a signal processing module; 120: a signal analysis module; 121: a baseline calculation unit; 122: a waveform comparison unit; 123: a determining unit; 130: an instruction reception module; 140: a signal input module; 150: a display module; 160: a storage module; and 170: a pattern selection module.

## DETAILED DESCRIPTION

Specific embodiments of the ablation lesion assessment system will be described in greater detail with reference to the accompanying drawings. Features and advantages of the invention will be more apparent from the following detailed description. Note that the figures are provided in a very simplified form not necessarily presented to scale, with their only intention to enhance convenience and clarity in explaining the disclosed embodiments. Furthermore, the terminology as used herein is for the purpose of describing the particular embodiments only and is not intended to be limiting of the invention. In this specification, singular references include the plural, unless the context clearly dictates otherwise. It will be further understood that the terms "comprises" and/or "comprising," when used in this specification, specify the presence of stated compounds, ingredients, components, steps, operations, and/or elements, but do not preclude the presence or addition of one or more other compounds, ingredients, components, steps, operations, and/or elements thereof.

In addition, components in the present invention, such as logically separable software (computer programs), hardware or equivalents thereof, are generally referred to as "units" or "modules". Therefore, "units" mentioned in the embodiments herein include both those in computer programs and those in hardware settings. Therefore, embodiments disclosed herein also include computer programs including instructions, which may be programs for carrying out each of steps in a computer, programs for implementing each computer function of as a tool, or programs for causing a computer to perform each of functions, systems or methods. "Units" or "modules" are activated by such computer programs. While the terms "storing", "stored" or equivalents thereof can be used herein for ease of description, they are meant to describe the storage of programs or the storage of computer programs in storage devices in a controlled manner. Although individual "modules" and "units" can be configured in substantial one-to-one correspondence with respective functions, in practice, a single module may be configured to have one or more programs, or multiple modules may be configured to have either a single program or multiple programs. In a distributed or parallel environment, multiple modules may be implemented by a single computer, or a single module may be implemented by multiple computers. A single module may include additional modules.

Herein, an "apparatus" and/or "system" may include multiple computers, hardware elements, devices or the like that are interconnected by communication appliances in an injection network with one-to-one connectivity, or a single computer, hardware element, device or the like capable of implementing a process in accordance with the present invention.

Further, each process, whether performed by a single module or unit, or by multiple modules or units, may involve reading required information from storage devices such as a memory device and writing the results from the process into the storage devices. Therefore, for each process described herein, a description of reading from and writing into storage devices prior and subsequent to the process may be omitted. Herein, "storage devices" may include hard drive disks, random access memory (RAM) devices, external storage media, storage devices accessible via communication connections, registers in central processing units (CPUs), etc.

In a radiofrequency ablation procedure, an ablation electrode attached to a distal end of an ablation catheter can emit RF energy for ablating a tissue so that a signal cannot propagate from one side of the ablated tissue to the other. The present invention proposes an ablation lesion assessment system, in which an intracardiac electrode signal from an electrode brought into contact with an ablation lesion is processed and analyzed to obtain real-time information indicating whether complete ablation of the lesion has been achieved. The proposed system can be used in cardiac radiofrequency ablation and provide at least the following two benefits. First, the intracardiac electrode signal from the ablation lesion can be graphically displayed to provide the surgeon with an indication of whether the lesion has been completely ablated, i.e., transmural. Second, the surgeon can be indicated in real time of the lesion's condition before, during and after the ablation. Accordingly, more reasonable ablation parameters can be set and personalized ablation strategies with associated ablation parameters can be provided for different heart places. The system, as well as its benefits, will be described in detail below by way of specific embodiments.

An ablation lesion assessment system 100 according to an embodiment of the present invention allows obtaining an intracardiac electrode signal from an electrode brought into contact with an ablation lesion (referred to hereinafter as the "electrode E", for the sake of clarity).

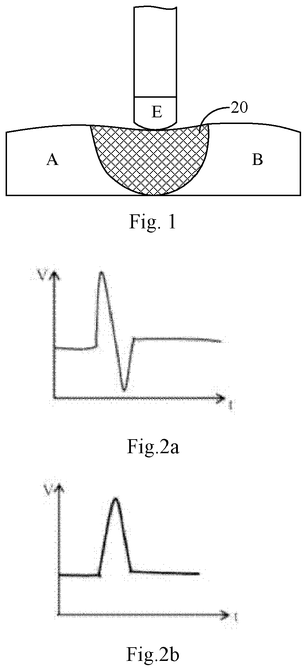

Specifically, the intracardiac electrode signal may be obtained by one or more electrodes brought into contact with the cardiac tissues. Preferably, the intracardiac electrode signal is obtained from one electrode that is disposed at a distal end of an ablation catheter or an intracardiac mapping catheter and brought into contact with a surface of the ablation lesion. That is, the intracardiac electrode signal is preferred to be a single-electrode signal. Fig. 1 schematically illustrates an electrode brought into contact with an ablation lesion in accordance with an embodiment of the present invention. Fig. 2(a) is a graphical representation of an intracardiac electrode signal from an incompletely ablated lesion in accordance with an embodiment of the present invention. Fig. 2(b) is a graphical representation of an intracardiac electrode signal from a completely ablated lesion in accordance with an embodiment of the present invention. Referring to Fig. 2(a), the intracardiac electrode signal obtained before complete ablation provides a voltage (V) signal profile over time (t) with a bipolar morphology with both positive portions corresponding to signal captured by the electrode E that is brought into contact with ablation lesion 20 from a side A of the electrode and negative portions corresponding to signal captured by the electrode E that is brought into contact with ablation lesion 20 from a side B of the electrode opposing the side A. With the ablation proceeding, the tissue at the ablation lesion 20 will become transmural and completely ablated, cutting off the path for interaction between electrical signals on both sides of the electrode E, as shown in Fig. 2(b), and the intracardiac electrode signal now exhibits a totally positive unipolar morphology.

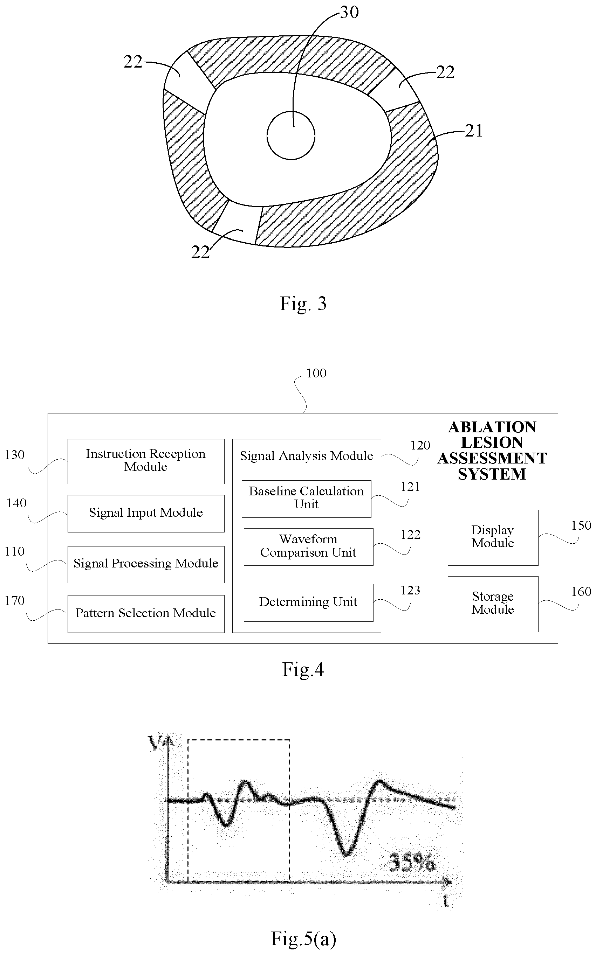

Using such a change in the intracardiac electrode signal fed back from the electrode E, assessment of ablation and transmurality is possible. With the use in pulmonary vein isolation (PVI) as an example, as schematically illustrated in Fig. 3, this procedure is required to form a continuous ablation circumferentially around each of one or more pulmonary veins 30. However, since the pulmonary vein 30 is complicated in anatomy and has different wall thicknesses at various sections, around the pulmonary veins 30, there may include both completely ablated transmural portions 21 and incompletely ablated nontransmural portions 22. The incompletely ablated nontransmural portions may lead to electrical reconnection and thus AF may recur or iatrogenic atrial arrhythmias may arise. Therefore, it is critical to control the ablation process so that the ablation lesion 20 is completely ablated and totally transmural without any gap therein. In accordance with embodiments of the present invention, an electrode E may be brought into contact with the ablation lesion 20 around the pulmonary vein 30 and an intracardiac electrode signal can be obtained therefrom. The signal profile of this intracardiac electrode signal can be constantly refreshed to enable real-time analysis and determination of whether a complete PVI has been obtained and whether the ablation is continuous.

An ablation lesion assessment system in accordance with an embodiment of the present invention will be described below in detail in the exemplary context of use in PVI.

This system is capable of processing the intracardiac electrode signal fed back from the electrode brought into contact with the ablation lesion and thereby obtaining ablation information. Fig. 4 shows the structure of the system. Referring to Figs. 1 and 4, the ablation lesion assessment system 100 includes:

a signal processing module 110 configured to process the intracardiac electrode signal fed back from the electrode that is brought into contact with the ablation lesion and thereby generate a signal profile (referred to hereinafter as the "signal profile L", for the sake of clarity); and

a signal analysis module 120 configured to analyze the signal profile L and thereby determine whether complete ablation has been achieved. To this end, the signal analysis module 120 may include a baseline calculation unit 121 and a determining unit 123 in some embodiments, or a waveform comparison unit 122 and the determining unit 123 in other embodiments, or the baseline calculation unit 121, the waveform comparison unit 122 and the determining unit 123 in still other embodiments. The signal analysis module is also configured to output ablation information indicating a result of the determination.

In some embodiments, the ablation lesion assessment system 100 may further include one or more of: an instruction reception module 130 for activating/deactivating the ablation lesion assessment system 100 and configuring parameters thereof; a signal input module 140 for obtaining the intracardiac electrode signal from the electrode E and making it available to the signal processing module 110; a display module 150 for displaying the signal profile L and the ablation information; a storage module 160 for storing ablation pattern(s) associated with the ablation lesion 20, as well as the signal profile L, data generated during operation of the ablation lesion assessment system 100 and the ablation information indicating whether complete ablation has been achieved; and a pattern selection module 170 for determining the intracardiac site where the ablation lesion 20 is being formed and selecting the associated ablation pattern(s) based on the intracardiac site.

The intracardiac electrode signal fed back from the electrode E may be time-dependent, and the signal processing module 110 may refresh and process the signal at a predetermined rate so that the generated signal profile L is always based upon real-time recording. The signal analysis module 120 may analyze the real-time signal profile L by running either or both of the baseline calculation unit 121 and the waveform comparison unit 122, and output the ablation information indicating whether complete ablation has been achieved.

The signal input module 140 may be a multi-channel electrophysiological recorder, a three-dimensional mapping system, a radiofrequency ablation system or other equipment capable of capturing the intracardiac electrode signal, e.g., as an electrocardiogram (ECG or EKG), a graph of voltage (V, ordinate) versus time (t, abscissa).

Upon receiving the intracardiac electrode signal from the signal input module 140, the signal processing module 110 may filter it at 0.05-300 Hz and refresh it at the aforementioned predetermined rate. The predetermined refresh rate may be 1-2000 Hz, with 1 Hz corresponding to one-second electrocardiogram segments being preferred because it is close to the human heartbeat rate.

The signal processing module 110 may then automatically identify a segment of the intracardiac electrode signal (e.g., electrocardiogram) fed back from the electrode E as the signal profile L. Apart from the automatic identification by the ablation lesion assessment system, the segment as the signal profile L may also be selected by the surgeon. The signal profile L may have two time endpoints that are associated with a heartbeat frequency reflected by the intracardiac electrode signal. For instance, a unipolar signal segment over one heartbeat cycle of the electrocardiogram may be identified or selected as the signal profile L. Alternatively, a segment between points respectively of a predetermined period of time (e.g., 200 ms) preceding and succeeding a peak in a heartbeat cycle may be identified or selected as the signal profile L. However, the present invention is not limited to this, because the segment as the signal profile L may be determined otherwise according to other methods known in the art.

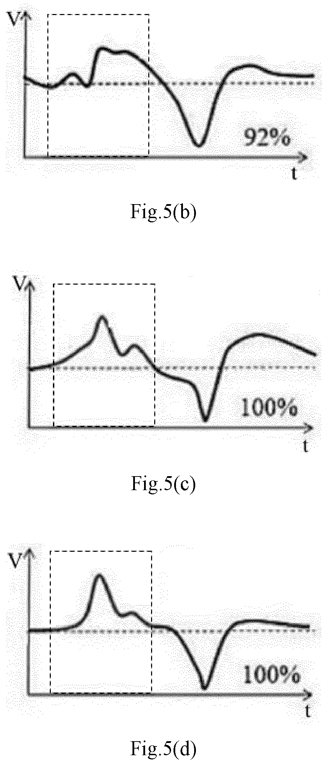

Figs. 5 (a) through 5 (d) are graphical representations of a signal profiles in an ablation process according to an embodiment of the present invention. Specifically, the four graphical representations of Figs. 5(a), 5(b), 5(c) and 5(d) are interceptions starting at respective time instants of an intracardiac electrode signal that evolves over time during the ablation process. In each of these figures, the signal profile L is encircled by a dashed box, and a dashed straight line marking a baseline for the signal profile L is shown. Operations of the baseline calculation unit 121 according to embodiments of the present invention will be explained in detail with reference to Figs. 5(a) to 5(d).

Analysis of the baseline calculation unit 121 on the signal profile L may involve: generating the baseline for the signal profile L by the baseline calculation unit 121; and calculating a proportion of positive portions located above the baseline in the whole signal profile L and feeding back the proportion to the determining unit 123.

Further, generating the baseline may include the steps of: (1) normalizing the signal profile L: firstly, collecting multiple adjacent sampling points (i.e. voltage value captured at certain time), the number of the sampling points depends on the sampling frequency, e.g., 512 per second at 512Hz, then defining a set of sampling points(e.g., 10) as a process window, hence to calculate the average value or weighted average value of each process window, thereby obtaining corresponding voltage value; (2) obtaining slopes by subtracting one of the voltage values of every adjacent two of the process windows from the other; and (3) setting the voltage value of the process window with the greatest slope as the baseline.

The generation of the baseline may also be accomplished with any of other common methods, such as finding the derivative of a fitted curve, wavelet transform, etc., a further detailed description of which is, however, omitted herein.

After the baseline has been generated for the signal profile L, a proportion of positive portions of the signal profile L located above the baseline in the whole signal profile L may be derived. This proportion can be calculated, e.g., as a ratio of the total area enclosed by the positive portions and the baseline to that enclosed by the whole signal profile L and the baseline. However, the present invention is not limited to this, as any form of the proportion of positive portions in the whole signal profile L is possible as long as it is obtained following the same convention.

In embodiments, the proportions of positive portions in the signal profiles L encircled in the respective dashed boxes in Figs. 5(a), 5(b) and 5(c) may be calculated as 35%, 92% and 100%, respectively. Fig. 5(d) shows a signal profile L delayed 5 seconds from the signal profile L of Fig. 5(c). The proportion may be output to the determining unit 123.

Next, operations of the waveform comparison unit 122 according to embodiments of the present invention will be explained in detail.

The waveform comparison unit 122 may analyze the signal profile L, and the analysis may include: obtaining a waveform comparison result through comparing the signal profile L with the ablation pattern(s) associated with the ablation lesion 20 by the waveform comparison unit 122; and feeding back the waveform comparison result to the determining unit 123.

The ablation lesion assessment system 100 may be prescribed or stored in the storage module 160 with one or more ablation patterns (referred to hereinafter as the "ablation pattern(s) M", for the sake of clarity). The ablation pattern(s) M may be, for example, signal profile(s) in successful ablation case(s). A single type of ablation lesion 20 (see Fig. 1) may be associated with one or more prescribed or stored ablation pattern(s) M. A comparison of the signal profile L with the ablation pattern(s) M by the waveform comparison unit 122 may result in a single waveform comparison result indicative of, for example, similarity or dissimilarity therebetween. If the waveform comparison result indicates similarity, then it is determined that the lesion has become transmural, i.e., completely ablated. The waveform comparison result may be output to the determining unit 123. Here, "similarity" means that the signal profile L has a pattern that is nearly the same as or identical to the ablation pattern(s) M.

For an ablation lesion 20 being created at an intracardiac site that is associated with a plurality of ablation patterns M, the waveform comparison unit 122 may calculate a Pearson correlation coefficient P (X, Y) between the signal profile L and each of the ablation patterns M according to

$$P(X,Y) = \frac{Cov(X,Y)}{\sigma_X \sigma_Y},$$

where X and Y denote X- and Y-coordinates associated with the signal profile and the ablation pattern M, $Cov(X,Y)$ is the covariance of X and Y, and $\sigma_X$ and $\sigma_Y$ are the standard deviations of X and Y The waveform comparison unit 122 may additionally obtain an overall Pearson correlation coefficient by taking the average or median of the resulting Pearson correlation coefficients. Specifically, if the overall Pearson correlation coefficient is less than 0.4, it may be determined that the signal profile L is weakly correlated or uncorrelated with the ablation patterns M. If the overall Pearson correlation coefficient is within the range of 0.4-0.6 (here, mathematically denoted as the interval [0.4, 0.6) or [0.4, 0.6]), it may be determined that the signal profile L is moderately correlated with the ablation patterns M. If the overall Pearson correlation coefficient is within the range of 0.6-1 (here, mathematically denoted as the interval [0.6, 1] or (0.6, 1], which continues with the foregoing interval without any overlap therewith), it may be determined that the signal profile L is strongly correlated with the ablation patterns M, satisfying the similarity criterion. In some embodiments, when the overall Pearson correlation coefficient is within the range of 0.6-1, the waveform comparison result indicates similarity. Otherwise, the waveform comparison result may indicate dissimilarity. Those of ordinary skill in the art will know how to calculate the Pearson correlation coefficients from publications, and a further detailed description thereof is omitted herein.

For an ablation lesion 20 being formed at an intracardiac site that is associated with a plurality of ablation patterns M, the waveform comparison unit 122 may also obtain a waveform comparison result by utilizing a neural network algorithm to compare the signal profile L with the ablation patterns M. In this case, the number of layers in the neural network is preferred to be equal to or greater than 10, and the ablation patterns M used in the neural network may be signal profiles in historical successful ablation cases, which have been calibrated by experts. The neural network may be trained with a set with a size preferably of greater than or equal to 5,000 signal profile patterns and then used to generate the output waveform comparison result. During the ablation process, the waveform comparison unit 122 may output one waveform comparison result to the determining unit 123 at every unit time interval (e.g., one second). The neural network algorithm may be any of existing algorithms known in the fields of pattern comparison and image processing, and a further detailed description thereof is omitted herein.

The waveform comparison unit 122 may also obtain the aforementioned waveform comparison result by using any of other suitable algorithms such as wavelet transform, speeded up robust features and Frechet distance.

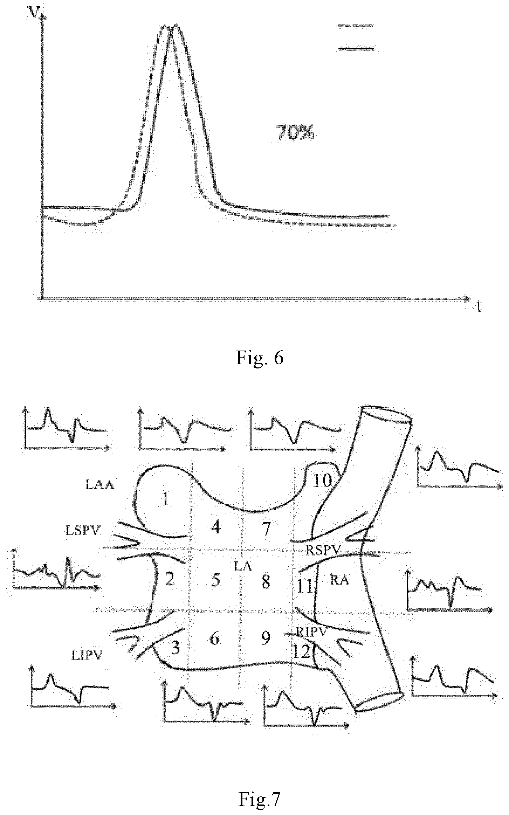

Fig. 6 schematically depicts a comparison drawn between a signal profile and an ablation pattern in accordance with an embodiment of the present invention. Referring to Figs. 1 and 6, the waveform comparison unit 122 may calculate a degree of waveform similarity between the signal profile L and the ablation pattern M associated with the ablation lesion 20. For example, the degree of waveform similarity between the signal profile L and the ablation pattern M associated with the ablation lesion 20 may be determined as a percentage such as 70%, or 100% when they coincides with each other. When the degree of waveform similarity is greater than a predetermined threshold (e.g., 80%, 90% or higher), the waveform comparison result output to the determining unit 123 may indicate similarity between the two. Otherwise, it will indicate dissimilarity therebetween. In order to obtain the degree of waveform similarity with higher accuracy, the signal profile L may be compared with each of available ablation patterns M and a statistical analysis may be then performed on the comparisons.

Those skilled in the art will appreciate that multiple intracardiac sites may need to be ablated in a single cardiac radiofrequency ablation procedure. With a PVI procedure as an example, it may be required to form a continuous ablation lesion circumferentially around each of one or more pulmonary veins. Preferably, according to an embodiment of the present invention, the storage module 160 in the ablation lesion assessment system 100 stores ablation patterns M for ablation lesions for various intracardiac sites. Moreover, the ablation lesion assessment system 100 may further include the pattern selection module 170 (see Fig. 4) for acquiring information about the intracardiac site where an ablation lesion 20 is being formed and selecting corresponding ablation pattern(s) M.

Fig. 7 schematically illustrates ablation lesions for different intracardiac sites and corresponding ablation patterns in accordance with embodiments of the present invention, in which, LAA : the left atrial appendage; LSPV: the left superior pulmonary vein; LIPV: the left inferior pulmonary vein; RSPV: the right superior pulmonary vein; RA: the right atrium; and RIPV: the right inferior pulmonary vein. Further, the pulmonary veins and the surrounding heart tissues may be divided into 12 subregions, in which the pulmonary veins are located in the sub-regions 5 and 8, while the other sub-regions (i.e., 1-4, 6, 7 and 9-12) form an annulus surrounding the pulmonary veins. In PVI procedures, ablation lesions are often formed in the surrounding sub-regions.

In preferred embodiments, during computational operations of the waveform comparison unit 122 for an ablation lesion 20, the pattern selection module 170 may first determine the intracardiac site where the ablation lesion 20 is being formed and then select ablation pattern(s) M associated with the intracardiac site. In this way, the waveform comparison unit 122 can compare a signal profile L from the electrode brought into contact with the ablation lesion 20 with the ablation pattern(s) M associated with the intracardiac site where the ablation lesion 20 is being formed and obtain a waveform comparison result. The pattern selection module 170 may be connected to a three-dimensional mapping system, which can automatically partition the atria and ventricles into sub-regions and automatically identify any of the sub-regions by a mapping catheter brought into contact therewith. With the aid of the three-dimensional mapping system, the pattern selection module 170 can acquire information about the target intracardiac site and select associated ablation pattern(s) M to which the signal profile L is compared by the waveform comparison unit 122. The functions of the three-dimensional mapping system can be performed by any suitable method known in the art, and a further detailed description thereof is omitted herein.

In summary, in accordance with embodiments of the present invention, the ablation lesion assessment system 100 may derive a proportion of positive portions of a signal profile L in the whole signal profile L from an analysis on the signal profile L by the baseline calculation unit 121, and transmit the proportion to the determining unit 123. Moreover, the ablation lesion assessment system 100 may obtain a waveform comparison result from a comparison drawn between the signal profile L and ablation pattern(s) M associated with the ablation lesion 20 by the waveform comparison unit 122, and transmit the waveform comparison result to the determining unit 123.

The ablation lesion assessment system 100 may choose either the baseline calculation unit 121 or the waveform comparison unit 122 to analyze the signal profile L, and provide the result to the determining unit 123. Alternatively, the baseline calculation unit 121 and the waveform comparison unit 122 may be chosen to analyze the signal profile L successively or simultaneously, and the respective results may be then sent to the determining unit 123.

Specifically, in some embodiments, upon receiving the proportion of positive portions in the whole signal profile L from the baseline calculation unit 121, the determining unit 123 may determine whether the proportion is equal to 100%, which indicates that the signal profile L is wholly above the baseline with all negative portions of the signal profile L having turned positive. If so, it may be determined that complete ablation has been achieved, and ablation information indicative of the success may be output. Otherwise, ablation information indicating that complete ablation has not been achieved may be output.

In other embodiments, when receiving the waveform comparison result between the signal profile L and the ablation pattern(s) M from the waveform comparison unit 122, the determining unit 123 may determine whether the waveform comparison result indicates similarity that means achieved complete ablation or dissimilarity that means unachieved complete ablation.

In some other embodiments, the determining unit 123 may make a determination based on both the proportion from the baseline calculation unit 121 and the waveform comparison result from the waveform comparison unit 122. In this case, ablation information indicating that complete ablation has been achieved may be output only when both of the proportion and waveform comparison result are satisfactory. Otherwise, ablation information indicating that complete ablation has not been achieved yet may be output.

The ablation information output from the determining unit 123 may be in the form of text. Moreover, all of the proportion, the degree of waveform similarity, the waveform comparison result and the ablation information may be displayed on the display module 150. For example, the ablation information output before the achievement of complete ablation may be displayed as "In Ablation", "Ablation in Progress" or "Ablation Not Completed", and that output after complete ablation has been achieved may be displayed as "Ablation Completed" or "Ablation Ceased". Alternatively, the ablation information output from the determining unit 123 may be in the form of a voice message saying, for example, "Ablation completed" or "Ablation Ceased" when the determining unit 123 determines that complete ablation has been achieved. In this case, the ablation lesion assessment system may also include a voice module, which is coupled to the determining unit and configured to play the voice message.

When determining that the proportion is equal to 100% and/or that the degree of waveform similarity is greater than a threshold, the determining unit 123 may output the ablation information indicating that complete ablation has been achieved to the display module 150 or voice module not immediately but after the elapse of (waiting for) a period of time which may be preconfigured at the instruction reception module 130 either manually or automatically. This can achieve the advantage of an enhanced probability of complete cell necrosis and a minimized chance of reversible damage. Here, "reversible damage" refers to damage that is created during a radiofrequency ablation procedure but is repaired later due to self-healing capability of the tissue. Reversible damage may lead to postoperative electrical reconnection and is therefore a condition that should be avoided as much as possible. The predetermined period of time may be, for example, 1-10 seconds or longer, preferably 2 seconds. It should be noted that if the predetermined period of time is too long, in addition to ablation lesion 20, it may cause excessive ablation and bring damage to other organs.

The ablation lesion assessment system 100 offers at least the following advantages.

First, the baseline calculation unit 121 is able to identify positive portions of a signal profile L and calculate a proportion of them in the whole signal profile L, which can be displayed to provide the surgeon with a convenient real-time indication of how the ablation is carrying on. In other words, when each negative wave of the signal profile L has turned positive, the surgeon can know that the ablation lesion is transmural. This can ensure both surgery validity and significantly enhanced safety of the procedure. Second, the waveform comparison unit 122 is able to compare the signal profile L with the ablation pattern(s) M in real time and thereby obtain a waveform comparison result, which also provides the surgeon a convenient real-time indication of how the ablation is carrying on. Third, it is preferred to compare multiple ablation patterns M with the real-time signal profile L and obtain the waveform comparison result as the average or median of the resulting Pearson correlation coefficients. Alternatively, the waveform comparison result may be obtained as a degree of waveform similarity between the signal profile L and multiple ablation patterns M that form a statistical sample group. This can minimize or avoid any error that may rise from the use of a single ablation pattern. Fourth, a waveform comparison result with higher accuracy can be obtained by using a neural network algorithm that has been trained with clinical unipolar electrocardiograms as the ablation patterns M. Fifth, in order to provide the surgeon with a more accurate indication, different ablation pattern(s) M may be chosen for intracardiac electrode signals from different intracardiac sites to obtain the waveform comparison result.

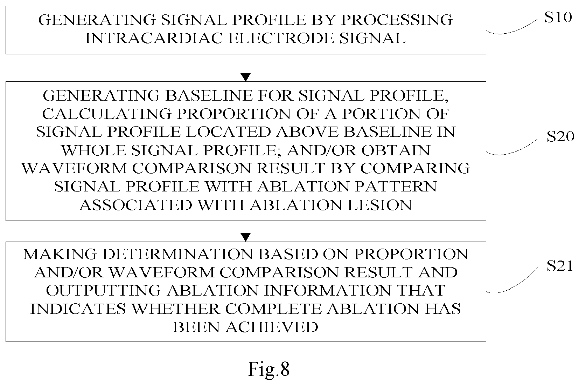

In accordance with examples not being claimed, there is also provided an ablation lesion assessment method for obtaining ablation information by processing an intracardiac electrode signal from an electrode brought into contact with the ablation lesion. Fig. 8 is a flowchart of this method, which includes the steps of:

S 10: generating a signal profile by processing the intracardiac electrode signal;

S20: generating a baseline for the signal profile and calculating a proportion of portions of the signal profile located above the baseline (i.e., positive portions) in the whole signal profile; and/or obtaining a waveform comparison result by comparing the signal profile with ablation pattern(s); and

S21: making a determination based on the proportion and/or the waveform comparison result and outputting ablation information that indicates whether complete ablation has been achieved.

This method can be implemented by the above-described system 100. For example, step S10 can be carried out by the signal processing module 110 in the system, while steps S20 and S21 can be performed by the signal analysis module 120.

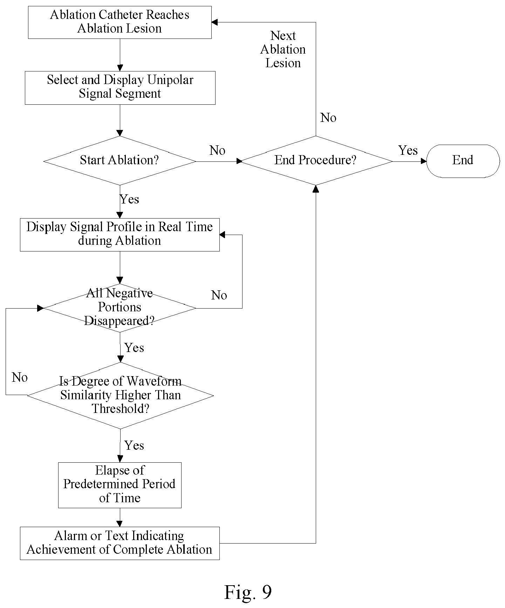

Fig. 9 schematically illustrates the use of the ablation lesion assessment method (not falling within the scope of the claims) in an ablation process in accordance with an example,

Referring to Fig. 9, for example, the ablation process may be a cardiac radiofrequency ablation procedure in which the electrode brought into contact with the surface of the ablation lesion is disposed at a distal end of an ablation catheter and the intracardiac electrode signal from the electrode experiences processes such as filtering and selection of a unipolar signal segment, resulting in the formation of the signal profile. At this point, it is determined whether to start ablation. If "Yes", the ablation catheter is activated to ablate the tissue, concurrently with the signal profile being refreshed at a predetermined rate and displayed. Specifically, a proportion of portions of the signal profile located above the baseline in the whole signal profile may be calculated, and when it reaches or exceeds 100% (i.e., all negative portions have disappeared or turned positive), a waveform comparison result may be obtained by comparing the signal profile with ablation pattern(s) associated with the ablation lesion. For example, a degree of waveform similarity may be calculated between the signal profile and the ablation pattern(s), and when this value is greater than a predetermined threshold, information indicating the achievement of complete ablation in the form of an alarm or text may be output after a predetermined period of time elapses. For example, the information indicating that complete ablation has been achieved may be displayed on a display device.

In this method, ablation information indicating whether complete ablation has been achieved can be obtained from analysis and processing of an intracardiac electrode signal from an electrode brought into contact with an ablation lesion and provided to the surgeon. For example, when all negative portions have turned positive, an indication of complete transmurality of the tissue being ablated may be provided. In this way, the reliance on the surgeon's experience can be minimized or eliminated, helping the surgeon know the progress of an intracardiac radiofrequency ablation procedure. Since this method has assessment features corresponding to those of the above-described ablation lesion assessment system, it has been described in a simplified way, and reference can be made to the above description of the ablation lesion assessment system for details in such assessment features. This method allows assessment of the information about the ablation lesion with which the electrode is brought into contact before, during and after ablation, thus resulting in a significantly increased success rate and safety of cardiac radiofrequency ablation.

Processing and execution in the foregoing embodiments are generally accomplished by the combination of a software program and hardware. However, they can also be implemented in part or wholly by electronic hardware. Whether by way of software or hardware, some portions are implementable to persons familiar with the fields of electronics and software and are thus not described in detail herein. The software program can be stored on a computer readable medium such as a CD-ROM or a memory in a computer system. When loaded into a computer, instructions of the software can be executed by a central processing unit (CPU).

The description presented above is merely that of some preferred embodiments of the present invention and does not limit the scope thereof in any sense. Any person skilled in the art can make possible changes and modifications to the subject matter of the present invention based on the above teachings. The invention is defined by the appended claims.

**Claims**

1. An ablation lesion assessment system (100) for obtaining an ablation information by processing and analyzing an intracardiac electrode signal from an ablation lesion, the ablation lesion assessment system (100) comprising:

   a signal processing module (110) configured to process the intracardiac electrode signal and to generate a signal profile, wherein the signal profile is a segment of the intracardiac electrode signal and is selected by a surgeon or automatic identified by the ablation lesion assessment system; and
   a signal analysis module (120) comprising a determining unit (123), wherein the signal analysis module (120) further comprises one or both of a baseline calculation unit (121) and a waveform comparison unit (122), wherein:

   the baseline calculation unit (121) is configured to generate a baseline for the signal profile, calculate a proportion of one or more positive portion of the signal profile located above the baseline to the whole signal profile and feed back the proportion to the determining unit (123),
   the waveform comparison unit (122) is configured to obtain a waveform comparison result by comparing the signal profile with an ablation pattern associated with the ablation lesion, and feed back the waveform comparison result to the determining unit (123), wherein the ablation pattern is a signal profile in successful ablation,
   the determining unit (123) is configured to make a determination based on the proportion and/or the waveform comparison result and output the ablation information that indicates whether a complete ablation has been achieved,
   wherein when the proportion is equal to 100%, the ablation information indicates that the complete ablation has been achieved, otherwise indicates that the complete ablation has not been achieved; or wherein when the waveform comparison result indicates a similarity, the ablation information indicates that the complete ablation has been achieved, otherwise indicates that the complete ablation has not been achieved, and
   wherein the waveform comparison unit (122) is configured to calculate a degree of waveform similarity between the signal profile and the ablation pattern associated with the ablation lesion, and when the degree of waveform similarity is greater than a predetermined threshold, the waveform comparison result indicates the similarity and otherwise indicates a dissimilarity.

2. The ablation lesion assessment system (100) of claim 1, wherein the intracardiac electrode signal varies over time, wherein the signal processing module (120) is configured to obtain and process the intracardiac electrode signal in real time at a predetermined refresh rate and thereby generate a corresponding real-time signal profile, and wherein the predetermined refresh rate is 1-2000 Hz.

3. The ablation lesion assessment system (100) of claim 1, wherein when the ablation information indicates that the complete ablation has been achieved, the determining unit (123) is configured to output the ablation information with a delay of a predetermined period of time after the determination is made.

4. The ablation lesion assessment system (100) of claim 2, wherein the predetermined period of time is 1-10 seconds.

5. The ablation lesion assessment system (100) of claim 1, wherein when the proportion is equal to 100%, the waveform comparison unit (122) feeds back the waveform comparison result to the determining unit (123), and when the waveform comparison unit (123) indicates a similarity, the ablation information indicates that the complete ablation has been achieved and otherwise indicates that the complete ablation has not been achieved.

6. The ablation lesion assessment system (100) of claim 1, wherein the ablation lesion is associated with a plurality of ablation patterns, and the waveform comparison unit (122) is configured to calculate a Pearson correlation coefficient between the signal profile and each of the plurality of ablation patterns and obtain an overall Pearson correlation coefficient by taking an average or a median of a plurality of Pearson correlation coefficients, wherein when the overall Pearson correlation coefficient is 0.6-1, the waveform comparison result indicates the similarity, otherwise indicates a dissimilarity, and wherein each of the plurality of Pearson correlation coefficients is

$$P(X,Y) = \frac{Cov(X,Y)}{\sigma_X \sigma_Y}$$

calculated according to ,
where P (X, Y) represents the Pearson correlation coefficient, X and Y denote X and Y coordinates of the signal profile and the ablation pattern, $Cov(X,Y)$ is a covariance of X and Y, and $\sigma_X$ and $\sigma_Y$ are standard deviations of X and Y.

7. The ablation lesion assessment system (100) of any one of claims 1 to 5, wherein the ablation lesion is associated with a plurality of ablation patterns, and the waveform comparison unit is configured to obtain the waveform comparison result by comparing the signal profile with the plurality of ablation patterns using a neural network algorithm.

8. The ablation lesion assessment system (100) of any one of claims 1 to 5, further comprising one or more of:

an instruction reception module (130) configured to activate or deactivate the ablation lesion assessment system (100) and to set parameters of the ablation lesion assessment system (100); a signal input module (140) configured to obtain and transmit the intracardiac electrode signal to the signal processing module (110); a display module (150) configured to display the ablation information; a voice module configured to convey the ablation information; a storage module (160) configured to store the ablation pattern; and a pattern selection module (170) configured to obtain an intracardiac site where the ablation lesion is being formed and to select an associated ablation pattern.

9. The ablation lesion assessment system (100) of any one of claims 1 to 5, wherein the intracardiac electrode signal is a single-electrode signal obtained by one electrode that is attached to an ablation catheter or an intracardiac mapping catheter and is brought into contact with the ablation lesion.

**Patentansprüche**

1. Ablationsläsionsbewertungssystem (100) zum Erhalten einer Ablationsinformation durch Verarbeiten und Analysieren eines intrakardialen Elektrodensignals von einer Ablationsläsion, wobei das Ablationsläsionsbewertungssystem (100) umfasst:

ein Signalverarbeitungsmodul (110), das ausgebildet ist, das intrakardiale Elektrodensignal zu verarbeiten und ein Signalprofil zu erzeugen, wobei das Signalprofil ein Segment des intrakardialen Elektrodensignals ist und von einem Chirurgen ausgewählt oder automatisch durch das Ablationsläsionsbewertungssystem identifiziert wird; und
ein Signalanalysemodul (120), umfassend eine Bestimmungseinheit (123), wobei das Signalanalysemodul (120) ferner eine oder beide von einer Basislinienberechnungseinheit (121) und einer Wellenformvergleichseinheit (122) umfasst, wobei:

die Basislinienberechnungseinheit (121) ausgebildet ist, eine Basislinie für das Signalprofil zu generieren, einen Anteil eines oder mehrerer positiver Abschnitte des Signalprofils, der über der Basislinie liegt, zu dem gesamten Signalprofil zu berechnen und den Anteil an die Bestimmungseinheit (123) zurückzumelden,
die Wellenformvergleichseinheit (122) ausgebildet ist, ein Wellenformvergleichsergebnis durch Vergleichen des Signalprofils mit einem mit der Ablationsläsion verknüpften Ablationsmuster zu erhalten, und das Wellenformvergleichsergebnis an die Bestimmungseinheit (123) zurückzumelden, wobei das Ablationsmuster ein Signalprofil bei erfolgreicher Ablation ist,
die Bestimmungseinheit (123) ausgebildet ist, eine Bestimmung basierend auf dem Anteil und/oder dem Wellenformvergleichsergebnis zu machen und die Ablationsinformation auszugeben, die angibt, ob eine vollständige Ablation erreicht wurde, wobei, wenn der Anteil gleich 100 % ist, die Ablationsinformation angibt, dass die vollständige Ablation erreicht wurde, und ansonsten, dass die vollständige Ablation nicht erreicht wurde; oder wobei, wenn das Wellenformvergleichsergebnis eine Ähnlichkeit angibt, die Ablationsinformation angibt, dass die vollständige Ablation erreicht wurde, und ansonsten, dass die vollständige Ablation nicht erreicht wurde, und wobei die Wellenformvergleichseinheit (122) ausgebildet ist, einen Grad der Wellenformähnlichkeit zwischen dem Signalprofil und dem mit der Ablationsläsion verknüpften Ablationsmuster zu berechnen, und wenn der Grad der Wellenformähnlichkeit größer als ein vorgegebener Schwellenwert ist, gibt das Wellenformvergleichsergebnis die Ähnlichkeit an und ansonsten eine Unähnlichkeit.

2. Ablationsläsionsbewertungssystem (100) nach Anspruch 1, wobei das intrakardiale Elektrodensignal zeitlich variiert, wobei das Signalverarbeitungsmodul (120) ausgebildet ist, das intrakardiale Elektrodensignal in Echtzeit mit einer vorgegebenen Bildwiederholfrequenz zu erhalten und dadurch ein entsprechendes Echtzeitsignalprofil zu erzeugen, und wobei die vorgegebene Bildwiederholfrequenz 1-2000 Hz ist.

3. Ablationsläsionsbewertungssystem (100) nach Anspruch 1, wobei, wenn die Ablationsinformation angibt, dass die vollständige Ablation erreicht wurde, die Bestimmungseinheit (123) ausgebildet ist, die Ablationsinformation mit einer Verzögerung um einen vorbestimmten Zeitraum, nachdem die Bestimmung gemacht wurde, auszugeben.

4. Ablationsläsionsbewertungssystem (100) nach Anspruch 2, wobei der vorgegebene Zeitraum 1-10 Sekunden ist.

5. Ablationsläsionsbewertungssystem (100) nach Anspruch 1, wobei, wenn der Anteil gleich 100 % ist, die Wellenformvergleichseinheit (122) das Wellenformvergleichsergebnis an die Bestimmungseinheit (123) zurückmeldet, und wenn die Wellenformvergleichseinheit (123) eine Ähnlichkeit angibt, gibt die Ablationsinformation an, dass die vollständige Ablation erreicht wurde, und ansonsten, dass die vollständige Ablation nicht erreicht wurde.

6. Ablationsläsionsbewertungssystem (100) nach Anspruch 1, wobei die Ablationsläsion mit einer Vielzahl von Ablationsmustern verknüpft ist, und wobei die Wellenformvergleichseinheit (122) ausgebildet ist, einen Pearson-Korrelationskoeffizienten zwischen dem Signalprofil und jedem der Vielzahl von Ablationsmustern zu berechnen und einen Gesamt-Pearson-Korrelationskoeffizienten durch Vornehmen eines Durchschnitt oder eines Median von einer Vielzahl von Pearson-Korrelationskoeffizienten zu erhalten, wobei, wenn der Gesamt-Pearson-Korrelationskoeffizient 0,6-1 ist, das Wellenformvergleichsergebnis die Ähnlichkeit angibt, und ansonsten eine Unähnlichkeit, und wobei jeder der Vielzahl von Pearson-Korrelationskoeffizienten gemäß

$$P(X,Y) = \frac{Cov(X,Y)}{\sigma_X \sigma_Y}$$ berechnet wird, wobei P (X, Y) den Pearson-Korrelationskoeffizienten darstellt, X und Y X- und Y-Koordinaten des Signalprofils und des Ablationsmuster bezeichnen, wobei $Cov(X,Y)$ eine Kovarianz von X und Y ist, und wobei $\sigma_X$ und $\sigma_Y$ Standardabweichungen von X und Y sind.

7. Ablationsläsionsbewertungssystem (100) nach einem der Ansprüche 1 bis 5, wobei die Ablationsläsion mit einer Vielzahl von Ablationsmustern verknüpft ist, und wobei die Wellenformvergleichseinheit ausgebildet ist, das Wellenformvergleichsergebnis durch Vergleichen des Signalprofils mit der Vielzahl von Ablationsmustern unter Verwendung eines neuronalen Netzwerkalgorithmus zu erhalten.

8. Ablationsläsionsbewertungssystem (100) nach einem der Ansprüche 1 bis 5, ferner umfassend ein oder mehrere von:

    einem Befehlsempfangsmodul (130), das ausgebildet ist, das Ablationsläsionsbewertungssystem (100) zu aktivieren oder zu deaktivieren, und Parameter des Ablationsläsionsbewertungssystems (100) einzustellen;
    einem Signaleingabemodul (140), das ausgebildet ist, das intrakardiale Elektrodensignal zu erhalten und an das Signalverarbeitungsmodul (110) zu übertragen;
    einem Anzeigemodul (150), das ausgebildet ist, die Ablationsinformation anzuzeigen;
    einem Sprachmodul, das ausgebildet ist, die Ablationsinformation zu übermitteln;
    einem Speichermodul (160), das ausgebildet ist, das Ablationsmuster zu speichern; und
    einem Musterauswahlmodul (170), das ausgebildet ist, eine intrakardiale Stelle zu erhalten, an der die Ablationsläsion gebildet wird, und ein zugehöriges Ablationsmuster auszuwählen.

9. Ablationsläsionsbewertungssystem (100) nach einem der Ansprüche 1 bis 5, wobei das intrakardiale Elektrodensignal ein Einzelelektrodensignal ist, das von einer Elektrode erhalten wird, die an einem Ablationskatheter oder einem intrakardialen Kartierungskatheter befestigt ist und mit der Ablationsläsion in Kontakt gebracht wird.

**Revendications**

1. Système d'évaluation de lésion d'ablation (100) pour obtenir des informations d'ablation par traitement et analyse d'un signal d'électrode intracardiaque provenant d'une lésion d'ablation, le système d'évaluation de lésion d'ablation (100) comprenant :

un module de traitement de signal (110) configuré pour traiter le signal d'électrode intracardiaque et générer un profil de signal, le profil de signal étant un segment du signal d'électrode intracardiaque et est sélectionné par un chirurgien ou identifié automatiquement par le système d'évaluation des lésions d'ablation ; et
un module d'analyse de signal (120) comprenant une unité de détermination (123), le module d'analyse de signal (120) comprenant en outre l'une ou les deux parmi une unité de calcul de ligne de base (121) et une unité de comparaison de forme d'onde (122), dans laquelle :

l'unité de calcul de ligne de base (121) est configurée pour générer une ligne de base pour le profil de signal, calculer une proportion d'une ou plusieurs parties positives du profil de signal située au-dessus de la ligne de base par rapport à l'ensemble du profil de signal et renvoyer la proportion à l'unité de détermination (123),
l'unité de comparaison de forme d'onde (122) est configurée pour obtenir un résultat de comparaison de forme d'onde en comparant le profil de signal avec un modèle d'ablation associé à la lésion d'ablation, et renvoyer le résultat de comparaison de forme d'onde à l'unité de détermination (123), le modèle d'ablation étant un profil de signal en cas d'ablation réussie,
l'unité de détermination (123) est configurée pour effectuer une détermination sur la base de la proportion et/ou du résultat de comparaison de forme d'onde et délivrer les informations d'ablation qui indiquent si une ablation complète a été réalisée,
dans lequel, lorsque la proportion est égale à 100 %, les informations d'ablation indiquent que l'ablation complète a été réalisée, sinon elles indiquent que l'ablation complète n'a pas été réalisée ; ou dans lequel, lorsque le résultat de la comparaison de forme d'onde indique une similarité, les informations d'ablation indiquent que l'ablation complète a été réalisée, sinon elles indiquent que l'ablation complète n'a pas été réalisée, et
dans lequel l'unité de comparaison de forme d'onde (122) est configurée pour calculer un degré de similarité de forme d'onde entre le profil de signal et le modèle d'ablation associé à la lésion d'ablation, et lorsque le degré de similarité de forme d'onde est supérieur à un seuil prédéterminé, le résultat de comparaison de forme d'onde indique la similitude et indique autrement une dissemblance.

2. Système d'évaluation de lésion d'ablation (100) selon la revendication 1, dans lequel le signal d'électrode intracardiaque varie dans le temps, dans lequel le module de traitement de signal (120) est configuré pour obtenir et traiter le signal d'électrode intracardiaque en temps réel à une fréquence de rafraîchissement prédéterminée et générer ainsi un profil de signal en temps réel correspondant, et dans lequel la fréquence de rafraîchissement prédéterminée est de 1 à 2 000 Hz.

3. Système d'évaluation de lésion d'ablation (100) selon la revendication 1, dans lequel lorsque les informations d'ablation indiquent que l'ablation complète a été réalisée, l'unité de détermination (123) est configurée pour émettre les informations d'ablation avec un retard d'une période de temps prédéterminée une fois la détermination effectuée.

4. Système d'évaluation de lésion d'ablation (100) selon la revendication 2, dans lequel la période de temps prédéterminée est de 1 à 10 secondes.

5. Système d'évaluation de lésion d'ablation (100) selon la revendication 1, dans lequel lorsque la proportion est égale à 100 %, l'unité de comparaison de forme d'onde (122) renvoie le résultat de la comparaison de forme d'onde à l'unité de détermination (123), et lorsque l'unité de comparaison de forme d'onde (123) 123) indique une similarité, les informations d'ablation indiquent que l'ablation complète a été réalisée et autrement indiquent que l'ablation complète n'a pas été réalisée.

6. Système d'évaluation de lésion d'ablation (100) selon la revendication 1, dans lequel la lésion d'ablation est associée à une pluralité de modèles d'ablation, et l' unité de comparaison de formes d'onde (122) est configurée pour calculer un coefficient de corrélation de Pearson entre le profil de signal et chacun des une pluralité de modèles d'ablation et obtenir un coefficient de corrélation de Pearson global en prenant une moyenne ou une médiane d'une pluralité de coefficients de corrélation de Pearson, dans lequel lorsque le coefficient de corrélation de Pearson global est de 0,6 à 1, le résultat de comparaison de forme d'onde indique la similarité, sinon indique une dissimilarité, et dans lequel chacun de la pluralité de coefficients de corrélation de Pearson est calculé

$$P(X,Y) = \frac{Cov(X,Y)}{\sigma_X \sigma_Y}$$

selon , où P (X, Y) représente le coefficient de corrélation de Pearson, X et Y désignent les coordonnées X et Y du profil de signal et du modèle d'ablation, $Cov(X,Y)$ est une covariance de X et Y, et $\sigma_X$ et $\sigma_Y$ sont des écarts types de X et Y.

7. Système d'évaluation de lésion d'ablation (100) selon l'une quelconque des revendications 1 à 5, dans lequel la lésion d'ablation est associée à une pluralité de modèles d'ablation, et l'unité de comparaison de forme d'onde est configurée pour obtenir le résultat de comparaison de forme d'onde en comparant le profil de signal avec le pluralité de modèles d'ablation à l'aide d'un algorithme de réseau neuronal.

8. Système d'évaluation de lésion d'ablation (100) selon l'une quelconque des revendications 1 à 5, comprenant en outre un ou plusieurs des éléments suivants :

> un module de réception d'instructions (130) configuré pour activer ou désactiver le système d'évaluation de lésion d'ablation (100) et pour définir des paramètres du système d'évaluation de lésion d'ablation (100) ;
> un module d'entrée de signal (140) configuré pour obtenir et transmettre le signal d'électrode intracardiaque au module de traitement de signal (110) ;
> un module d'affichage (150) configuré pour afficher les informations d'ablation ;
> un module vocal configuré pour transmettre les informations d'ablation ;
> un module de stockage (160) configuré pour stocker le modèle d'ablation ; et
> un module de sélection de modèle (170) configuré pour obtenir un site intracardiaque où la lésion d'ablation est en cours de formation et pour sélectionner un modèle d'ablation associé.

9. Système d'évaluation de lésion par ablation (100) selon l'une quelconque des revendications 1 à 5, dans lequel le signal d'électrode intracardiaque est un signal à électrode unique obtenu par une électrode qui est fixée à un cathéter d'ablation ou à un cathéter de cartographie intracardiaque et est mise en contact avec la lésion d'ablation.

Fig. 1

Fig.2a

Fig.2b

Fig. 3

Fig.4

Fig.5(a)

Fig.5(b)

Fig.5(c)

Fig.5(d)

Fig. 6

Fig.7

| GENERATING SIGNAL PROFILE BY PROCESSING INTRACARDIAC ELECTRODE SIGNAL | S10 |
|---|---|

↓

| GENERATING BASELINE FOR SIGNAL PROFILE, CALCULATING PROPORTION OF A PORTION OF SIGNAL PROFILE LOCATED ABOVE BASELINE IN WHOLE SIGNAL PROFILE; AND/OR OBTAIN WAVEFORM COMPARISON RESULT BY COMPARING SIGNAL PROFILE WITH ABLATION PATTERN ASSOCIATED WITH ABLATION LESION | S20 |
|---|---|

↓

| MAKING DETERMINATION BASED ON PROPORTION AND/OR WAVEFORM COMPARISON RESULT AND OUTPUTTING ABLATION INFORMATION THAT INDICATES WHETHER COMPLETE ABLATION HAS BEEN ACHIEVED | S21 |
|---|---|

Fig.8

Ablation Catheter Reaches
Ablation Lesion

Next
Ablation
Lesion

Select and Display Unipolar
Signal Segment

No

Start Ablation? — No → End Procedure? — Yes → End

Yes

Display Signal Profile in Real Time
during Ablation

All Negative
Portions
Disappeared? — No

Yes

Is Degree of Waveform
Similarity Higher Than
Threshold?

No

Yes

Elapse of
Predetermined Period
of Time

Alarm or Text Indicating
Achievement of Complete Ablation

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3212074 A1

- US 2004059237 A1